# EUROPEAN PATENT SPECIFICATION

(11) **EP 3 501 632 B1**
(45) Date of publication and mention of the grant of the patent: **10.08.2022**
(21) Application number: 18206947.6
(22) Date of filing: 19.11.2018
(51) Int. Cl.: B01J 7/00, C10L 3/08, B01J 12/00, C07C 1/12

(54) **APPARATUS OF PRODUCING METHANE AND METHOD FOR PRODUCING METHANE USING THE SAME**
VORRICHTUNG ZUR HERSTELLUNG VON METHAN UND VERFAHREN ZUR HERSTELLUNG VON METHAN DAMIT
APPAREIL DE PRODUCTION DE MÉTHANE ET PROCÉDÉ DE PRODUCTION DE MÉTHANE Y FAISANT APPEL

(30) Priority: 18.12.2017 JP 2017241970
(43) Date of publication of application: 26.06.2019
(73) Proprietor: Kabushiki Kaisha Toyota Chuo Kenkyusho, Nagakute-shi, Aichi 480-1192 (JP)
(72) Inventor: ITOH, Yoshihiko, Nagakute-shi, Aichi 480-1192 (JP); SAKAI, Masatoshi, Nagakute-shi, Aichi 480-1192 (JP); IMAGAWA, Haruo, Nagakute-shi, Aichi 480-1192 (JP); SAYAMA, Shogo, Nagakute-shi, Aichi 480-1192 (JP); BABA, Naoki, Nagakute-shi, Aichi 480-1192 (JP)
(74) Representative: Kramer Barske Schmidtchen Patentanwälte PartG mbB

(56) References cited:
- EP-A1- 2 843 031
- EP-A2- 2 216 094
- WO-A1-2016/007825
- CN-A- 103 666 611

## Description

### BACKGROUND OF THE INVENTION

### Field of the Invention

The present invention relates to an apparatus of producing methane and a method for producing methane using the same, in particular to an apparatus of producing methane from a CO₂-containing gas and a method for producing methane from a CO₂-containing gas using the same.

### Related Background Art

Methods for producing methane using CO₂ as a raw material and using a methanation catalyst such as Ru or Ni have been drawing attention in terms of the reduction of CO₂ emissions in the global warming problems. However, production of methane which uses as a raw material a gas containing CO₂ and O₂ such as combustion exhaust gas or biogas makes it necessary to increase the purity of CO₂ upstream of the methanation catalyst and remove reaction inhibiting components such as O₂. Additionally, practically-used examples of the method for producing methane using a CO₂-containig gas as a raw material include a method which individually combines a CO₂ separation and recovery apparatus (chemical adsorption method, physical adsorption method, etc.) with a methanation catalyst apparatus. However, the method requires heat when separating and recovering CO₂. This makes it necessary to supply external energy to the apparatuses and poses a problem of complex and large apparatuses.

In light of the above, Japanese Unexamined Patent Application Publication No. Hei 5-193920 (Patent Document 1) proposes a method which precipitates carbon on the ferrite surface by passing CO₂ and H₂ through the ferrite within a temperature range of 300°C to 400°C, and then converts the carbon, precipitated on the ferrite, to methane by raising the temperature of the ferrite to 600°C in H₂ without the supply of CO₂. However, although this method makes it possible to precipitate carbon from CO₂ within a temperature range of 300°C to 400°C, it is necessary to provide external energy because this method requires raising the temperature to 600°C or more in the methanation of the carbon. Moreover, it is necessary to remove O₂ in advance prior to supplying CO₂ to the ferrite.

Additionally, Japanese Unexamined Patent Application Publication No. Hei 9-110731 (Patent Document 2) proposes a method for producing methane which alternately repeats the carbon dioxide decomposition step of bringing a CO₂-containig gas into contact with an active ferritic iron oxide obtained by removing oxygen in a ferrite-based iron oxide crystal and the methane production step of bringing an H₂-containing gas into contact with the active ferritic iron oxide obtained in the carbon dioxide decomposition step. However, this method requires removing O₂ in advance prior to bringing the CO₂-containing gas into contact with the active ferritic iron oxide. In addition, it is difficult to continuously produce methane using a CO₂-containing gas as a raw material.

Furthermore, International Publication No. WO2006/007825 (Patent Document 3), Applied Catalysis B: Environmental, 168 and 169 (2015), 370 to 376 (Non-Patent Document 1), and Catalysts, 2017, 7, 88 (Non-Patent Document 2) propose a method which allows a CO₂ storage-reduction catalyst, having CO₂ storage capacity and methane generation ability, to store CO₂ and then supplies H₂ to reduce the stored CO₂ for conversion into methane. Since this method is capable of separation/recovery of CO₂ and methanation reaction with a single catalyst, it is possible to simplify and downsize the apparatus. Furthermore, since this method is capable of using heat generated in the methanation reaction for separation/recovery of CO₂, it is possible to separate/recover CO₂ and to carry out methanation reaction at a temperature of 250 to 400°C without supplying external energy. However, in the methods of Patent Document 3 and Non-Patent Documents 1 and 2, it is difficult to continuously produce methane using a CO₂-containing gas as a raw material because the methods require alternate separation/recovery of CO₂ and methanation reaction. EP 2 843 031 A1 discloses an apparatus for cogeneration of gas-steam based on gasification and methanation of biomass comprising two primary reactors connected in parallel and secondary methanation reactors. EP 2 216 094 A2 discloses CO₂-sorptive pellets and uses thereof.

### SUMMARY OF THE INVENTION

The present inventors have found a problem of the methods described in Patent Document 3 and Non-Patent Documents 1 and 2 that, in supplying a purge gas to remove oxygen in the catalytic reactor after the storage of CO₂ or in supplying H₂ for reduction reaction, the stored CO₂ is desorbed from the catalyst, resulting in the decrease in the concentration and the purity of methane generated.

The present invention has been made in view of the above problem of the conventional techniques, and an object thereof is to provide an apparatus and a method for producing methane which can continuously produce methane using a CO₂-containing gas as a raw material and which can further enhance the concentration and the purity of methane in the methane-containing gas to be obtained.

Specifically, the object is achieved by an apparatus producing methane from a CO₂-containing gas according to claim 1 or a method for producing methane from a CO₂-containing gas according to claim 4. Further improvements are given in the dependent claims.

The present inventors have made earnest studies for the purpose of achieving the above object and completed the present invention as a result. The findings are as follows: It is possible to continuously produce methane using a CO₂-containing gas as a raw material when two or more reactors provided with a CO₂ storage-reduction catalyst are disposed in parallel, each of the reactors alternately stores CO₂ into the CO₂ storage-reduction catalyst and reduces the stored CO₂, and at least one of the reactors stores CO₂ and simultaneously a remaining reactor reduces the stored CO₂. In addition, it is possible to prevent emission of CO₂ to the outside of the system by supplying a purge gas, supplied to the reactor after the storage of CO₂, to the reactor after the reduction reaction and by storing the CO₂ contained in the purge gas into the CO₂ storage-reduction catalyst after the reduction reaction. Furthermore, it is possible to enhance the concentration and the purity of methane in the methane-containing gas to be obtained by reducing the CO₂, contained in the reaction-produced gas obtained by reduction of the CO₂, using a methanation catalyst disposed downstream.

The present invention makes it possible to continuously produce methane using a CO₂-containing gas as a raw material and further to enhance the concentration and the purity of methane in the methane-containing gas to be obtained.

### BRIEF DESCRIPTION OF THE DRAWINGS

FIG. 1 is a schematic diagram illustrating a preferred embodiment of an apparatus of producing methane of the present invention.
FIG. 2 is a schematic diagram illustrating another preferred embodiment of the apparatus of producing methane of the present invention.
FIG. 3 is a schematic diagram illustrating another preferred embodiment of the apparatus of producing methane of the present invention.
FIG. 4 is a schematic diagram illustrating another preferred embodiment of the apparatus of producing methane of the present invention.
FIG. 5 is a schematic diagram illustrating another preferred embodiment of the apparatus of producing methane of the present invention.
FIG. 6A is a flowchart for a reactor A1 which is provided with a CO₂ storage-reduction catalyst in the production of methane carried out in Example 1.
FIG. 6B is a flowchart for a reactor A2 which is provided with a CO₂ storage-reduction catalyst in the production of methane carried out in Example 1.
FIG. 6C is a flowchart for a reactor B which is provided with a methanation catalyst in the production of methane carried out in Example 1.
FIG. 7A is a flowchart for a gas storage container C1 and the reactor A1 provided with a CO₂ storage-reduction catalyst in the production of methane carried out in Example 2.
FIG. 7B is a flowchart for a gas storage container C2 and the reactor A2 provided with a CO₂ storage-reduction catalyst in the production of methane carried out in Example 2.
FIG. 7C is a flowchart for the reactor B provided with a methanation catalyst in the production of methane carried out in Example 2.
FIG. 8 is a graph illustrating the amount of CO₂ removed per hour in the production of methane carried out in Examples 1 and 2 and Comparative Examples 1 and 2.
FIG. 9 is a graph illustrating the amount of CO₂ emitted per hour in the production of methane carried out in Examples 1 and 2 and Comparative Examples 1 and 2.
FIG. 10 is a graph illustrating the amount of methane generated per hour in the production of methane carried out in Examples 1 and 2 and Comparative Examples 1 and 2.
FIG. 11 is a schematic diagram illustrating an apparatus of producing methane used in Comparative Example 1.
FIG. 12 is a schematic diagram illustrating an apparatus of producing methane used in Comparative Example 2.

### DETAILED DESCRIPTION OF THE PREFERRED EMBODIMENTS

Hereinafter, preferred embodiments of the present invention will be described in detail with reference to the drawings, but the present invention is not limited to the drawings. Note that in the following description and drawings, the same or equivalent elements are denoted by the same reference numerals, and redundant explanations may be omitted.

First, a description is provided for a CO₂ storage-reduction catalyst, a methanation catalyst, a CO₂-containing gas, a reducing gas, and a purge gas used in the present invention.

The CO₂ storage-reduction catalyst is a catalyst having a CO₂ storage capacity and a methane generation ability. Examples thereof include a catalyst supporting a component having a methanation catalytic ability and a component having a CO₂ storage capacity on a catalytic support. The component having the methanation catalytic ability is not particularly limited, and examples thereof include Ru, Ni, Pt, Pd, Rh, Co, Fe, and Mn. These methanation catalytic components may be used singly or used in combination of two or more kinds. The component having the CO₂ storage capacity is not particularly limited, and examples thereof include alkali metal compounds, alkaline earth metal compounds, and rare earth compounds. These components having the CO₂ storage capacity may be used singly or used in combination of two or more kinds. In addition, the components having the methanation catalytic ability and the components having the CO₂ storage capacity are supported on a catalytic support (more preferably, a porous catalytic support). The component constituting the catalytic support is preferably alumina, silica, silica-alumina, titania, zirconia, ceria, ceria-zirconia, and the like.

The methanation catalyst has a catalytic ability of generating methane by reducing CO₂. Examples thereof include catalysts which support the methanation catalytic component such as Ru, Ni, Pt, Pd, Rh, Co, Fe, and Mn on the porous catalytic support.
These methanation catalysts may be used singly or used in combination of two or more kinds.

The CO₂-containing gas is not particularly limited as long as it contains CO₂. However, the CO₂-containing gas is preferably a gas at least containing CO₂ and O₂ from the viewpoint that use of the production apparatus and the production method for methane of the present invention makes it possible to continuously produce methane using the CO₂-containing gas as a raw material and further to enhance the concentration and the purity of methane in the methane-containing gas to be obtained.

The reducing gas is not particularly limited as long as it can reduce CO₂ to methane. Examples thereof include H₂ gas, NH₃ gas, hydrazine gas, and a gas containing these gases. In addition, examples of the purge gas include noble gases such as He and inert gases such as N₂.

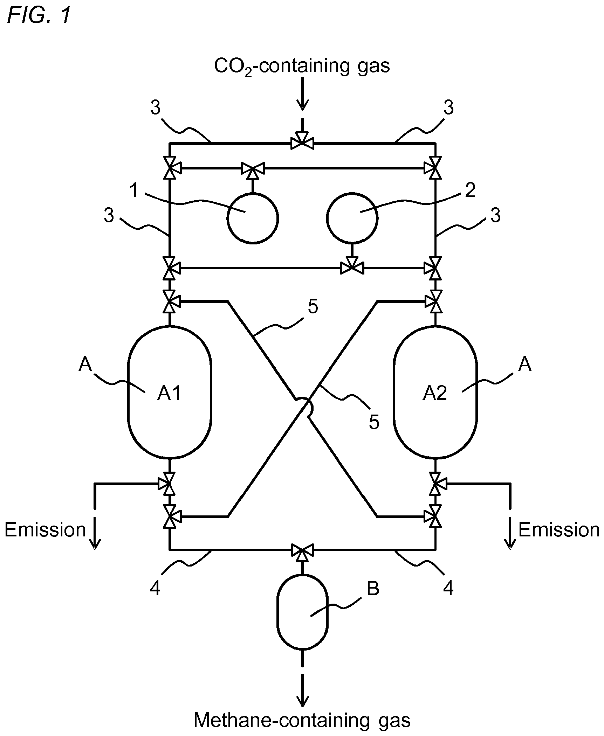

Next, a description is provided for the apparatus of producing methane of the present invention. FIG. 1 to FIG. 5 are each a schematic diagram illustrating a preferred embodiment of the apparatus of producing methane of the present invention. As illustrated in FIG. 1 to FIG. 5, the apparatus of producing methane of the present invention is an apparatus of producing methane from a CO₂-containing gas, including a reactor A which is provided with the CO₂ storage-reduction catalyst having the CO₂ storage capacity and the methane generation ability, a reactor B which is provided with the methanation catalyst, a means 1 for supplying purge gas, and a means 2 for supplying reducing gas.

The reactor A provided with the CO₂ storage-reduction catalyst used in the present invention is not particularly limited. Examples thereof include a catalytic reactor packed with powder of the CO₂ storage-reduction catalyst, a catalytic reactor packed with pellets of the CO₂ storage-reduction catalyst, and a catalytic reactor packed with a honeycomb or foam coated with the CO₂ storage-reduction catalyst. In the apparatus of producing methane of the present invention, two or more of the reactors A provided with the CO₂ storage-reduction catalyst as described above are disposed in parallel. For example, in the apparatuses of producing methane of the present invention illustrated in FIG. 1 to FIG. 5, two reactors A provided with the CO₂ storage-reduction catalyst are disposed in parallel. In addition, the apparatus of producing methane of the present invention is not limited to this. Three or more reactors A provided with the CO₂ storage-reduction catalyst may be disposed in parallel. By disposing two or more reactors A provided with the CO₂ storage-reduction catalyst in parallel as described above, it is possible to alternately store and reduce CO₂ in each of the reactors A, and to store CO₂ in some reactors A and simultaneously to reduce CO₂ in the remaining reactors A, making it possible to continuously produce methane using the CO₂-containing gas as a raw material. Moreover, the gas inlet of each of the two or more reactors A provided with the CO₂ storage-reduction catalyst is connected to a corresponding gas supply line 3 for supplying the CO₂-containing gas. Furthermore, the gas outlet of each of the reactors A provided with the CO₂ storage-reduction catalyst is connected to a corresponding gas transfer line 4 for transferring the reaction-produced gas obtained in the reactors A provided with the CO₂ storage-reduction catalyst to a reactor B provided with the methanation catalyst.

The reactor B provided with the methanation catalyst used in the present invention is not particularly limited. Examples thereof include a catalytic reactor packed with powder of the methanation catalyst, a catalytic reactor packed with pellets of the methanation catalyst, and a catalytic reactor packed with a honeycomb or foam coated with the methanation catalyst. In the apparatus of producing methane of the present invention, at least one reactor B provided with the methanation catalyst as described above is disposed downstream of the reactors A provided with the CO₂ storage-reduction catalyst on the gas flow paths, specifically, the gas transfer lines 4 are connected to the gas inlet of the reactor B provided with the methanation catalyst. For example, in the apparatuses of producing methane of the present invention illustrated in FIG. 1 to FIG. 5, one reactor B provided with the methanation catalyst is disposed downstream of the reactors A provided with the CO₂ storage-reduction catalyst on the gas flow paths. In addition, the apparatus of producing methane of the present invention is not limited to this. Two or more reactors B provided with the methanation catalyst may be disposed as long as they are disposed downstream of the reactors A provided with the CO₂ storage-reduction catalyst on the gas flow paths. By disposing at least one reactor B provided with the methanation catalyst downstream of the reactors A provided with the CO₂ storage-reduction catalyst on the gas flow paths as described above, it is possible to generate methane by reducing CO₂ contained in the reaction-produced gas obtained in the reactors A provided with the CO₂ storage-reduction catalyst and to enhance the concentration and the purity of methane in the methane-containing gas to be obtained.

No particular limitation is imposed on the means 1 for supplying purge gas used in the present invention. Examples thereof include a gas cylinder filled with the purge gas, an N₂ gas production unit which removes oxygen from the atmosphere using a porous film, a separation membrane or a freezer, and a unit for gasifying liquid nitrogen. In the apparatus of producing methane of the present invention, at least one means 1 for supplying purge gas as described above is disposed upstream of the reactors A provided with the CO₂ storage-reduction catalyst on the gas flow paths (specifically, at least one means 1 for supplying purge gas is connected to the gas supply line 3). For example, in the apparatuses of producing methane of the present invention illustrated in FIG. 1 to FIG. 5, one means 1 for supplying purge gas is disposed upstream of the reactors A provided with the CO₂ storage-reduction catalyst on the gas flow paths (specifically, one means 1 for supplying purge gas is connected to all of the gas supply lines 3). In addition, the apparatus of producing methane of the present invention is not limited to this. Two or more means 1 for supplying purge gas may be each disposed upstream of the reactors A provided with the CO₂ storage-reduction catalyst on two or more gas flow paths (specifically, the means 1 for supplying purge gas may be each connected to the gas supply lines 3 which are connected to the respective two or more reactors A provided with the CO₂ storage-reduction catalyst).

No particular limitation is imposed on the means 2 for supplying reducing gas used in the present invention. Examples thereof include a gas cylinder filled with the reducing gas, a water electrolyzer, a pyrolysis apparatus for ammonia or methylcyclohexane, an H₂ gas generation unit which desorbs hydrogen from a hydrogen storage material, and a unit for gasifying liquid hydrogen. In the apparatus of producing methane of the present invention, at least one means 2 for supplying reducing gas as described above is disposed upstream of the reactors A provided with the CO₂ storage-reduction catalyst on the gas flow paths (specifically, at least one means 2 for supplying reducing gas is connected to the gas supply line 3). For example, in the apparatuses of producing methane of the present invention illustrated in FIG. 1 to FIG. 5, one means 2 for supplying reducing gas is disposed upstream of the reactors A provided with the CO₂ storage-reduction catalyst on the gas flow paths (specifically, one means 2 for supplying reducing gas is connected to all of the gas supply lines 3). In addition, the apparatus of producing methane of the present invention is not limited to this. Two or more means 2 for supplying reducing gas may be each disposed upstream of the reactors A provided with the CO₂ storage-reduction catalyst on two or more gas flow paths (specifically, the means 2 for supplying reducing gas may be each connected to the gas supply lines 3 which are connected to the respective two or more reactors A provided with the CO₂ storage-reduction catalyst).

Furthermore, in the apparatus of producing methane of the present invention, the gas outlet of a reactor A provided with the CO₂ storage-reduction catalyst and the gas inlet of at least one different reactor A, other than that reactor, provided with the CO₂ storage-reduction catalyst are connected together via a purge gas recirculation line 5 which supply the purge gas emitted from the gas outlet of the reactor A into the gas inlet of the different reactor A. For example, in the apparatuses of producing methane of the present invention each of which includes the two reactors A1 and A2 each provided with the CO₂ storage-reduction catalyst, as illustrated in FIG. 1 to FIG. 5, the gas outlet of the reactor A1 and the gas inlet of the reactor A2 are connected together via the purge gas recirculation line 5, and the gas outlet of the reactor A2 and the gas inlet of the reactor A1 are connected together via the purge gas recirculation line 5. In addition, in an apparatus of producing methane of the present invention which includes three reactors A1, A2, and A3 each provided with the CO₂ storage-reduction catalyst (not illustrated), the gas outlet of the reactor A1 and the gas inlet of the reactor A2 are connected together via the purge gas recirculation line 5, the gas outlet of the reactor A2 and the gas inlet of the reactor A3 are connected together via the purge gas recirculation line 5, and the gas outlet of the reactor A3 and the gas inlet of the reactor A1 are connected together via the purge gas recirculation line 5. The connection between the gas outlet of an arbitrary reactor A provided with the CO₂ storage-reduction catalyst and the gas inlet of a different reactor A provided with the CO₂ storage-reduction catalyst via the purge gas recirculation line 5 as described above makes it possible to prevent emission of CO₂ to the outside of the system by supplying the purge gas emitted from the reactor A after the storage of CO₂ to the reactor A after the reduction reaction and then allowing the CO₂ storage-reduction catalyst to store the CO₂ contained in the purge gas during the removal of reaction-inhibiting components such as O₂ by supplying a purge gas to a reactor A after the storage of CO₂. As a result, it is possible to enhance the concentration and the purity of methane in the methane-containing gas to be obtained.

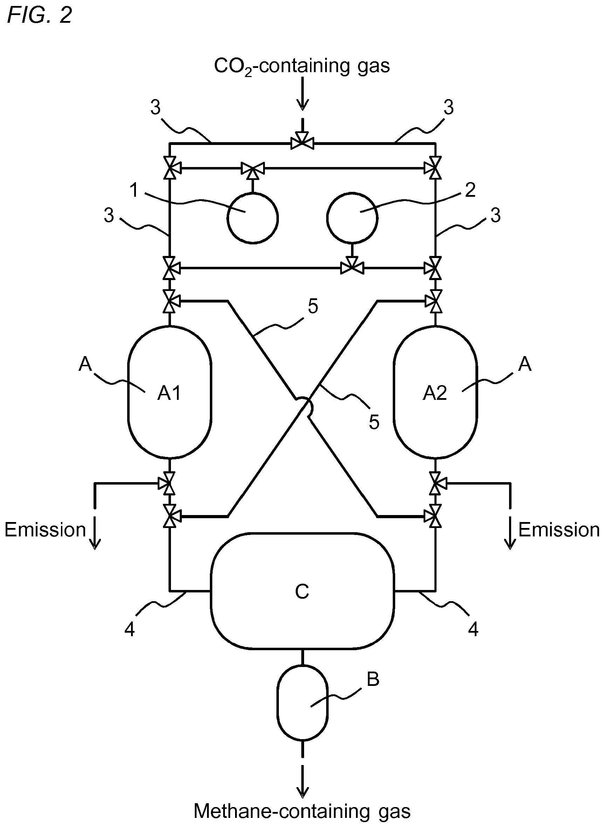

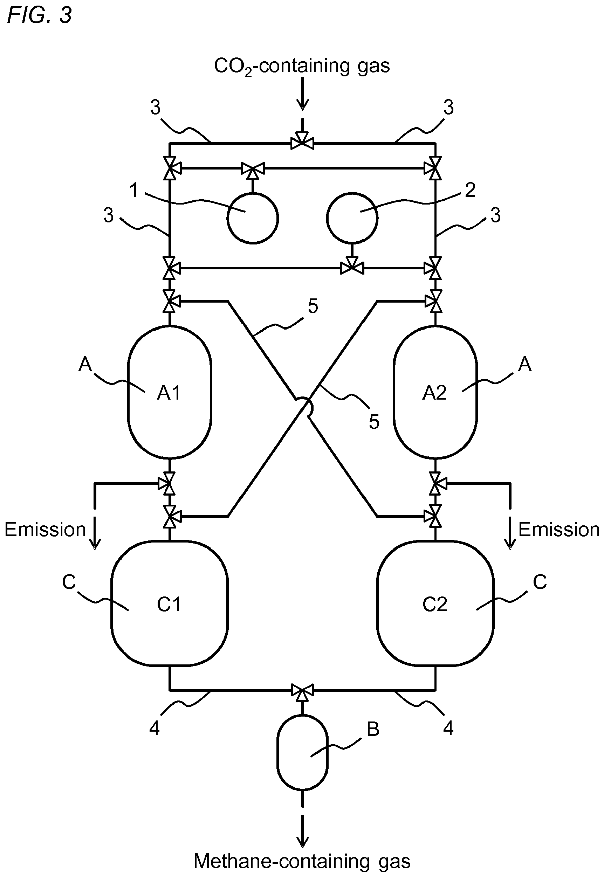

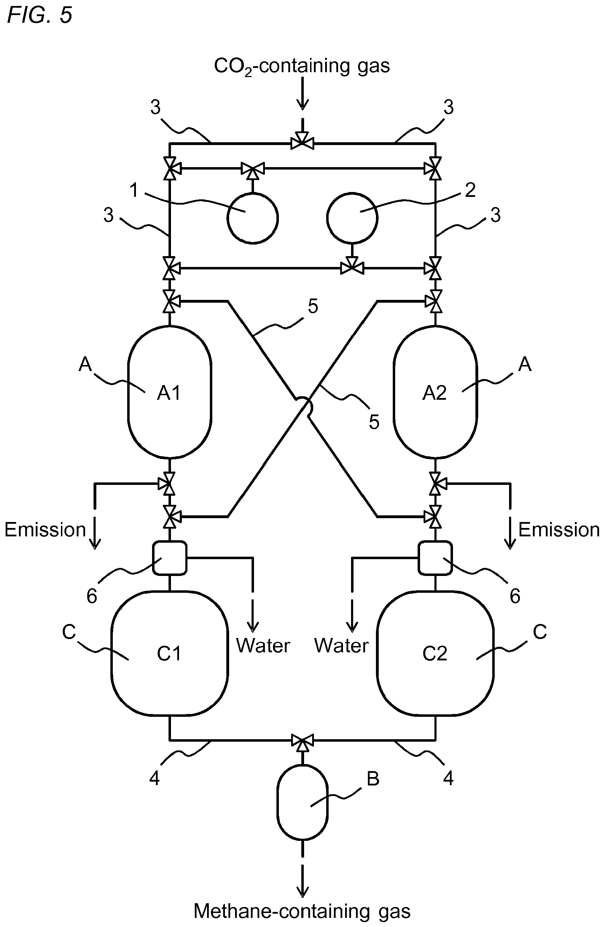

In addition, in the apparatus of producing methane of the present invention, at least one gas storage container C for storing the reaction-produced gas emitted from the gas outlets of the reactors A provided with the CO₂ storage-reduction catalyst is further preferably disposed between the reactors A provided with the CO₂ storage-reduction catalyst and the reactor B provided with the methanation catalyst (specifically, in the gas transfer lines 4). For example, in the apparatus of producing methane of the present invention illustrated in FIG. 2, one gas storage container C is disposed between the reactors A provided with the CO₂ storage-reduction catalyst and the reactor B provided with the methanation catalyst (specifically, in the gas transfer lines 4). In the apparatuses of producing methane of the present invention illustrated in FIG. 3 and FIG. 5, two gas storage containers C are disposed between the reactors A provided with the CO₂ storage-reduction catalyst and the reactor B provided with the methanation catalyst (specifically, in the gas transfer lines 4). Additionally, the apparatus of producing methane of the present invention is not limited to these. Three or more gas storage containers C may be disposed between the reactors A provided with the CO₂ storage-reduction catalyst and the reactor B provided with the methanation catalyst (specifically, in the gas transfer lines 4). This makes it easy to control the reaction-produced gas to be supplied to the reactor B provided with the methanation catalyst and to supply at a suitable ratio the desorbed CO₂ and unreacted H₂ to the reactor B provided with the methanation catalyst. Thus, it is possible to further enhance the concentration and the purity of methane in the methane-containing gas to be obtained.

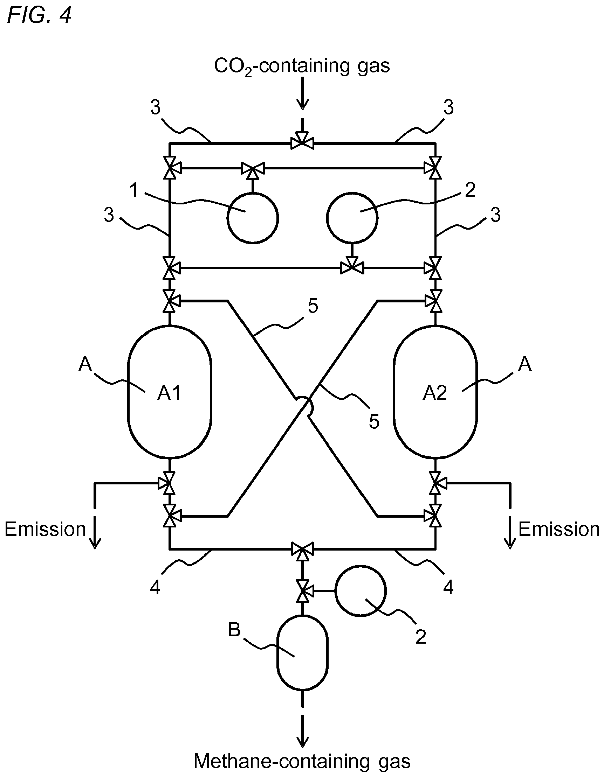

Moreover, in the apparatus of producing methane of the present invention, at least one means 2 for supplying reducing gas is further preferably disposed between the reactors A provided with the CO₂ storage-reduction catalyst and the reactor B provided with the methanation catalyst (specifically, in the gas transfer line 4). For example, in the apparatus of producing methane of the present invention illustrated in FIG. 4, one means 2 for supplying reducing gas is disposed between the reactors A provided with the CO₂ storage-reduction catalyst and the reactor B provided with the methanation catalyst (specifically, in the gas transfer line 4). In addition, the apparatus of producing methane of the present invention is not limited to this. Two or more means 2 for supplying reducing gas may be disposed between the reactors A provided with the CO₂ storage-reduction catalyst and the reactor B provided with the methanation catalyst (specifically, in the gas transfer lines 4). This makes it possible to supply at a suitable ratio CO₂ and the reducing gas to the reactor B provided with the methanation catalyst. Thus, it is possible to further enhance the concentration and the purity of methane in the methane-containing gas to be obtained.

In addition, in the apparatus of producing methane of the present invention, at least one means 6 for removing moisture in gas is further preferably disposed between the reactors A provided with the CO₂ storage-reduction catalyst and the reactor B provided with the methanation catalyst (specifically, in the gas transfer line 4). For example, in the apparatus of producing methane of the present invention illustrated in FIG. 5, two means 6 for removing moisture in gas are disposed between the reactors A provided with the CO₂ storage-reduction catalyst and the gas storage containers C (specifically, in the gas transfer lines 4). In addition, the apparatus of producing methane of the present invention is not limited to this. One or three or more means 6 for removing moisture in gas may be disposed between the reactors A provided with the CO₂ storage-reduction catalyst and the reactor B provided with the methanation catalyst (specifically, in the gas transfer lines 4). This removes water, an inhibitory factor of methanation reaction, and enhances methanation activity. In addition, the apparatus of producing methane of the present invention illustrated in FIG. 5 can reduce the amount of reaction-produced gas supplied to the reactor B provided with the methanation catalyst. This makes it possible to reduce the volume of the gas storage container C and thus to downsize the apparatus.

Next, a description is provided for the method for producing methane of the present invention. The method for producing methane of the present invention is a method for producing methane from the CO₂-containing gas using the apparatus of producing methane of the present invention, comprising the steps of: allowing the CO₂ storage-reduction catalyst to store CO₂ by supplying the CO₂-containing gas to the reactor A provided with the CO₂ storage-reduction catalyst [CO₂ storage step]; reducing the CO₂ by supplying the reducing gas to the reactor A provided with the CO₂ storage-reduction catalyst having stored the CO₂ [CO₂ reduction step]; supplying a reaction-produced gas obtained by reduction of the CO₂ to the reactor B provided with the methanation catalyst [reaction-produced gas supply step]; supplying a purge gas to the reactor A after the storage of the CO₂ and supplying the purge gas emitted from the reactor A to the reactor A after the reduction reaction [purge gas recirculation step]; and reducing residual CO₂ in the reaction-produced gas supplied to the reactor B provided with the methanation catalyst [methanation reaction step].

In addition, in the method for producing methane of the present invention, each of the reactors A provided with the CO₂ storage-reduction catalyst alternately carries out the CO₂ storage step and the CO₂ reduction step, and at least one of the reactors A provided with the CO₂ storage-reduction catalyst carries out the CO₂ storage step and simultaneously a remaining reactor A provided with the CO₂ storage-reduction catalyst carries out the CO₂ reduction step.

Moreover, in the method for producing methane of the present invention, it is preferable to store the reaction-produced gas obtained by reduction of CO₂ in the gas storage container C and then to supply the reaction-produced gas to the reactor B provided with the methanation catalyst.

Hereinafter, a description is provided for the method for producing methane of the present invention taking as an example the case of using the apparatuses of producing methane illustrated in FIG. 1 to FIG. 5 each of which includes two reactors A1 and A2 provided with the CO₂ storage-reduction catalyst. However, the method for producing methane of the present invention is not limited to these.

The method for producing methane of the present invention supplies the CO₂-containing gas to the reactor A1 provided with the CO₂ storage-reduction catalyst after the reduction reaction and simultaneously allows the means 1 for supplying purge gas to supply the purge gas to the reactor A2 provided with the CO₂ storage-reduction catalyst after the storage of CO₂. This allows the reactor A1 to store the CO₂ contained in the CO₂-containing gas into the CO₂ storage-reduction catalyst and allows the reactor A2 to remove the reaction-inhibiting components such as O₂. In addition, the purge gas emitted from the reactor A2 is also supplied to the reactor A1 via the purge gas recirculation line 5. This allows the reactor A1 to store the CO₂ which is contained in the purge gas emitted from the reactor A2 into the CO₂ storage-reduction catalyst, making it possible to prevent emission of CO₂ to the outside of the system and to enhance the concentration and the purity of methane in the methane-containing gas to be obtained.

Next, the reactor A1 is supplied only with the CO₂-containing gas, and simultaneously, the reactor A2 is supplied with a reducing gas from the means 2 for supplying reducing gas. This subsequently allows the reactor A1 to store the CO₂ contained in the CO₂-containing gas into the CO₂ storage-reduction catalyst and allows the reactor A2 to reduce the CO₂ stored in the CO₂ storage-reduction catalyst. Consequently, a reaction-produced gas, containing methane, CO₂ desorbed from the CO₂ storage-reduction catalyst, and an unreacted reducing gas, is obtained. This reaction-produced gas is supplied via the gas transfer lines 4 to the reactor B provided with the methanation catalyst. In the reactor B, the residual CO₂ is reduced for production of methane. This enhances the concentration and the purity of methane in the methane-containing gas to be obtained.

Next, the reactor A2 after the reduction reaction is supplied with a purge gas from the means 1 for supplying purge gas to remove reaction-inhibiting components such as O₂ in the reactor A2. After that, the reactor A2 is supplied with the CO₂-containing gas, and simultaneously, the reactor A1 after the storage of CO₂ is supplied with the purge gas from the means 1 for supplying purge gas. This allows the reactor A2 to store the CO₂ contained in the CO₂-containing gas into the CO₂ storage-reduction catalyst and allows the reactor A1 to remove the reaction-inhibiting components such as O₂. In addition, the purge gas emitted from the reactor A1 is also supplied to the reactor A2 via the purge gas recirculation line 5. This allows the reactor A2 to store the CO₂ which is contained in the purge gas emitted from the reactor A1 into the CO₂ storage-reduction catalyst, making it possible to prevent emission of CO₂ to the outside of the system and to enhance the concentration and the purity of methane in the methane-containing gas to be obtained.

Next, the reactor A2 is supplied only with the CO₂-containing gas, and simultaneously, the reactor A1 is supplied with the reducing gas from the means 2 for supplying reducing gas. This subsequently allows the reactor A2 to store the CO₂ contained in the CO₂-containing gas into the CO₂ storage-reduction catalyst and allows the reactor A1 to reduce the CO₂ stored in the CO₂ storage-reduction catalyst. Consequently, a reaction-produced gas, containing methane, CO₂ desorbed from the CO₂ storage-reduction catalyst, and an unreacted reducing gas, is obtained. This reaction-produced gas is also supplied via the gas transfer lines 4 to the reactor B provided with the methanation catalyst. In the reactor B, the residual CO₂ is reduced for production of methane. This enhances the concentration and the purity of methane in the methane-containing gas to be obtained.

Once the method for producing methane of the present invention repeats the series of operations described above, the reactors A1 and A2 provided with the CO₂ storage-reduction catalyst alternately repeat the CO₂ storage step and the CO₂ reduction step, making it possible to continuously produce methane using the CO₂-containing gas as a raw material.

In addition, in the method for producing methane of the present invention, it is preferable to store the reaction-produced gas obtained in the reactors A1 and A2 into the gas storage container C by using the apparatuses of producing methane provided with the gas storage container C illustrated in FIG. 2, FIG. 3, and FIG. 5 and then to supply the reaction-produced gas to the reactor B. This makes it easy to control the reaction-produced gas to be supplied to the reactor B and to supply at a suitable ratio the desorbed CO₂ and unreacted H₂ to the reactor B. Thus, it is possible to further enhance the concentration and the purity of methane in the methane-containing gas to be obtained.

Moreover, in the method for producing methane of the present invention, it is preferable to supply a reducing gas to the reaction-produced gas obtained in the reactors A1 and A2 by using the apparatus of producing methane illustrated in FIG. 4 which includes the means 2 for supplying the reducing gas to the reactor B provided with the methanation catalyst. This makes it possible to supply at a suitable ratio CO₂ and the reducing gas to the reactor B, further enhancing the concentration and the purity of methane in the methane-containing gas to be obtained.

In addition, in the method for producing methane of the present invention, it is preferable to remove moisture, an inhibitory factor of methanation reaction, from the reaction-produced gas obtained in the reactors A1 and A2 by using the apparatus of producing methane provided with the means 6 for removing moisture in gas, illustrated in FIG. 5. This enhances the methanation activity and also makes it possible to reduce the amount of reaction-produced gas supplied to the reactor B. Consequently, it is possible to reduce the volume of the gas storage container C and thus to downsize the apparatus.

In the method for producing methane of the present invention, no particular limitation is imposed on the method for controlling the steps described above. For example, the supply gases may be switched over time, or the supply gases may be switched when a predetermined gas concentration is reached, with the gas concentration measured with a gas sensor attached to the gas outlet of the reactor A, reactor B, or the gas storage container C.

As above, a description has been provided for preferred embodiments of the method for producing methane of the present invention taking as an example the case of using the apparatus of producing methane which includes two reactors provided with the CO₂ storage-reduction catalyst. However, the method for producing methane of the present invention is not limited to the embodiments described above. For example, also in the case of using an apparatus of producing methane which includes three or more reactors provided with the CO₂ storage-reduction catalyst, it is possible to continuously produce methane using the CO₂-containing gas as a raw material when these reactors are used in parallel, each of the reactors alternately stores CO₂ into the CO₂ storage-reduction catalyst and reduces the stored CO₂, and at least one of the reactors stores CO₂ and simultaneously a remaining reactor reduces the stored CO₂. In addition, it is possible to prevent emission of CO₂ to the outside of the system by supplying the purge gas, supplied to the reactor after the storage of CO₂, to the reactor after the reduction reaction and then storing the CO₂ contained in the purge gas into the CO₂ storage-reduction catalyst after the reduction reaction. Furthermore, it is possible to enhance the concentration and the purity of methane in the methane-containing gas to be obtained by reducing the CO₂, contained in the reaction-produced gas obtained by reduction of the CO₂, using the methanation catalyst disposed downstream.

### [Examples]

Hereinafter, the present invention is described more specifically based on Examples and Comparative Examples. However, the present invention is not limited to the examples below.

### (Example 1)

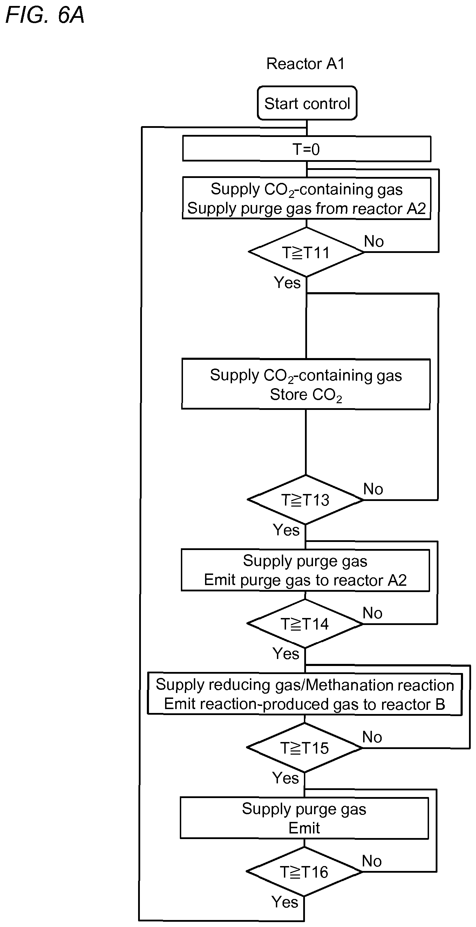

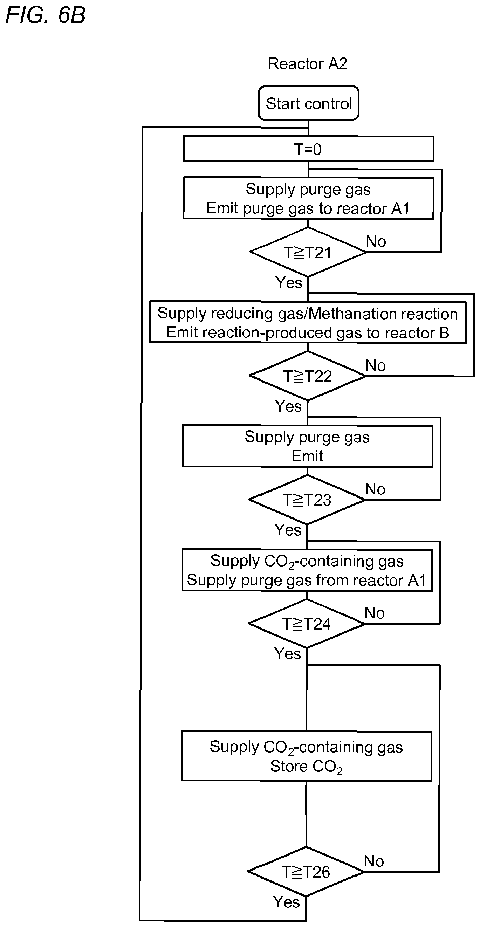

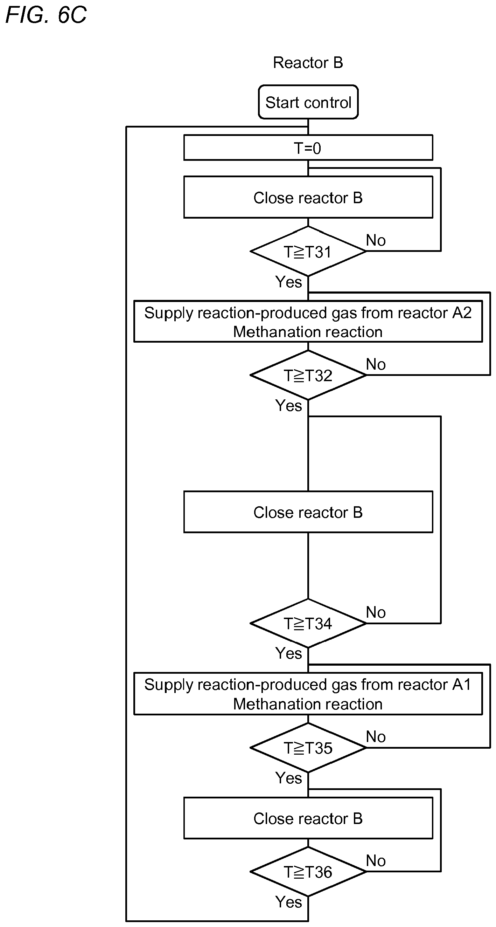

Methane was continuously produced with a gas containing CO₂ and O₂ as a raw material by using the apparatus of producing methane illustrated in FIG. 1 and in accordance with the flowcharts described in FIG. 6A to FIG. 6C and with the time settings described in Table 1. In the apparatus of producing methane illustrated in FIG. 1, two reactors A1 and A2 provided with a CO₂ storage-reduction catalyst were disposed in parallel. The gas inlet of each of the reactors A1 and A2 was connected to the gas supply line 3 for supplying a CO₂-containing gas. The gas supply line 3 was connected to the means 1 for supplying purge gas and the means 2 for supplying reducing gas. In addition, the gas outlet of each of the reactors A1 and A2 was connected to the gas transfer line 4 for transferring the obtained reaction-produced gas to the reactor B provided with a methanation catalyst. Moreover, the gas outlet of the reactor A1 and the gas inlet of the reactor A2 were connected together via the purge gas recirculation line 5. Simultaneously, the gas outlet of the reactor A2 and the gas inlet of the reactor A1 were connected together via the purge gas recirculation line 5.

The CO₂ storage-reduction catalyst used was 2 g of catalyst supporting 10% by mass of calcium acetate in terms of CaO and 5% by mass of Ru on an alumina catalytic support. In addition, the methanation catalyst used was 1 g of a commercially available methanation catalyst supporting 2% by mass of Ru in terms of metal Ru on titania. The temperatures of the reactors A1 and A2 and the reactor B were set to 320°C. The CO₂-containing gas used was a mixture gas of 10% of CO₂ + 3% of O₂ + He (balance), and its flow rate was set to 20 ml/min. The purge gas used was a 100% He gas, and its flow rate was set to 20 ml/min. The reducing gas used was a 100% H₂ gas, and its flow rate was set to 10.5 ml/min.

**[Table 1]**

| Time [sec] | Reactor A1 | Reactor A2 | Reactor B |
|---|---|---|---|
| 7.5 | T11 | T21 | T31 |
| 140.5 | --- | T22 | T32 |
| 148.0 | T13 | T23 | --- |
| 155.5 | T14 | T24 | T34 |
| 288.5 | T15 | --- | T35 |
| 296.0 | T16 | T26 | T36 |

As shown in FIG. 6A to FIG. 6C and Table 1, the reactor A1 after the reduction reaction (excluding the first cycle) was supplied with the CO₂-containing gas for 7.5 seconds, and simultaneously, the reactor A2 after the storage of CO₂ (excluding the first cycle) was supplied with the purge gas for 7.5 seconds. In addition, in this period of time (for 7.5 seconds), the purge gas emitted from the reactor A2 was supplied to the reactor A1 via the purge gas recirculation lines 5. This allowed the reactor A1 to store CO₂ into the CO₂ storage-reduction catalyst and allowed the reactor A2 to remove reaction-inhibiting components such as O₂. Note that the reactor B was closed in this period of time.

Next, methane was generated (excluding the first cycle) by supplying the reactor A2 with the reducing gas for 133 seconds to reduce the CO₂ stored in the CO₂ storage-reduction catalyst. A methane-containing gas was obtained (excluding the first cycle) by supplying the reactor B with the obtained reaction-produced gas (considered to contain methane as well as CO₂ desorbed from the CO₂ storage-reduction catalyst and an unreacted reducing gas) and reducing the residual CO₂ in the reactor B for generation of methane. Note that the reduction time of CO₂ in the reactor B was 133 seconds, and the reactor B was closed after the emission of the methane-containing gas. After that, the reactor A2 was supplied with the purge gas for 7.5 seconds followed by emission as it was. On the other hand, in this period of time (140.5 seconds), the reactor A1 was supplied only with the CO₂-containing gas to store CO₂ in the CO₂ storage-reduction catalyst.

Next, the reactor A2 was supplied with the CO₂-containing gas for 7.5 seconds, and simultaneously, the reactor A1 after the storage of CO₂ was supplied with the purge gas for 7.5 seconds. In addition, the purge gas emitted from the reactor A1 was supplied to the reactor A2 via the purge gas recirculation lines 5. This allowed the reactor A2 to store CO₂ into the CO₂ storage-reduction catalyst and allowed the reactor A1 to remove the reaction-inhibiting components such as O₂. Note that the reactor B was successively closed (15 seconds in total).

Next, methane was generated by supplying the reactor A1 with the reducing gas for 133 seconds to reduce the CO₂ stored in the CO₂ storage-reduction catalyst. A methane-containing gas was obtained by supplying the reactor B with the obtained reaction-produced gas (considered to contain methane as well as CO₂ desorbed from the CO₂ storage-reduction catalyst and an unreacted reducing gas) and reducing the residual CO₂ in the reactor B for generation of methane. Note that the reduction time of CO₂ in the reactor B was 133 seconds, and the reactor B was closed after the emission of the methane-containing gas. After that, the reactor A1 was supplied with the purge gas for 7.5 seconds followed by emission as it was. On the other hand, in this period of time (140.5 seconds), the reactor A2 was supplied only with the CO₂-containing gas to store CO₂ in the CO₂ storage-reduction catalyst.

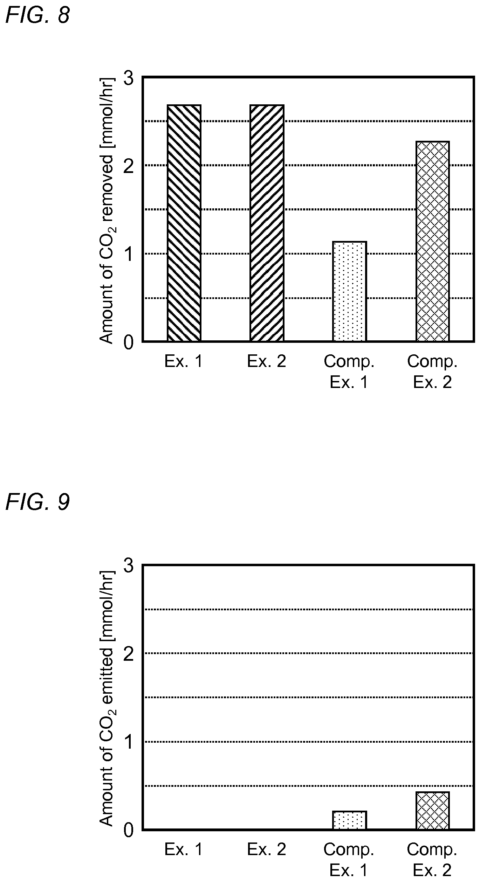

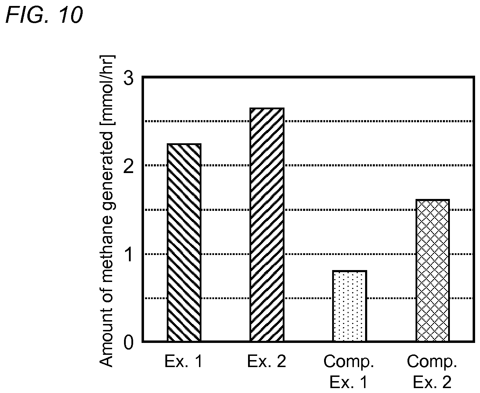

The series of operations described above was repeated until a steady state was reached. A mass spectrometer was used to measure the CO₂ concentrations and methane concentrations in the incoming gas (CO₂-containing gas) and the outgoing gas (methane-containing gas) into/from the apparatus in the steady state. Determined were the amount of CO₂ removed, the amount of CO₂ emitted, and the amount of methane generated per hour based on the obtained measurement results. Table 3 and FIG. 8 to FIG. 10 show the results.

### (Example 2)

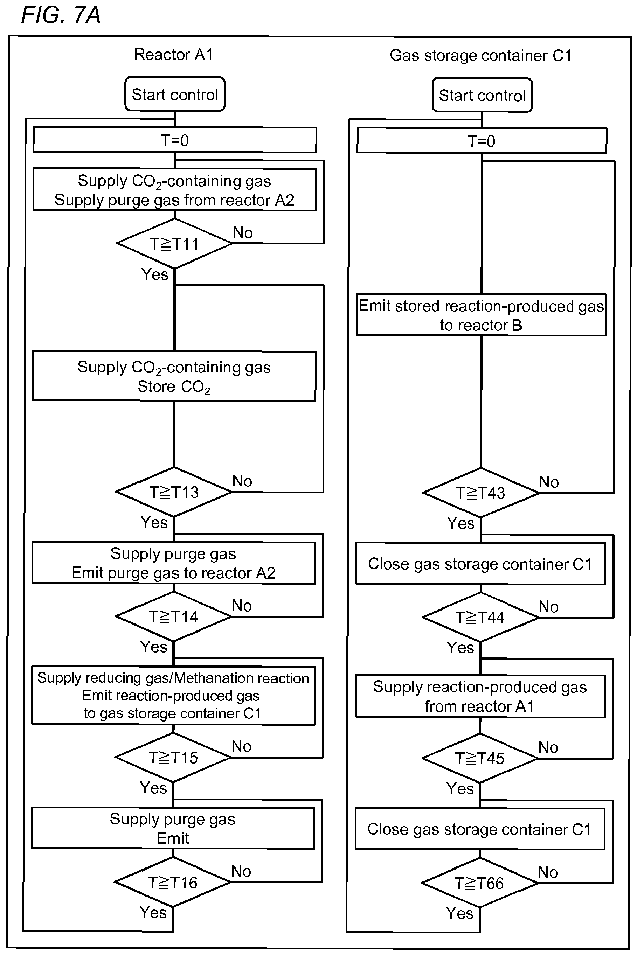

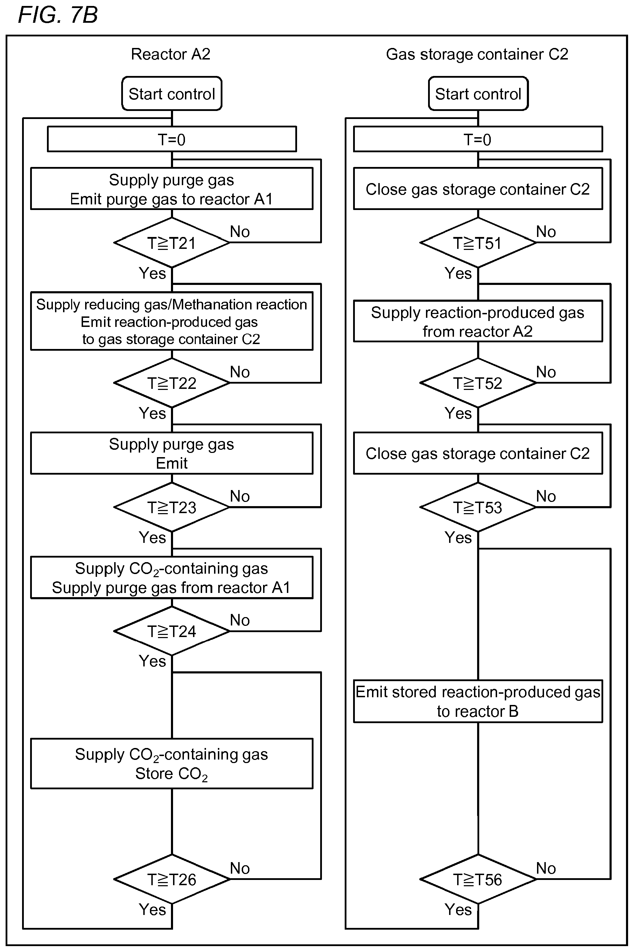

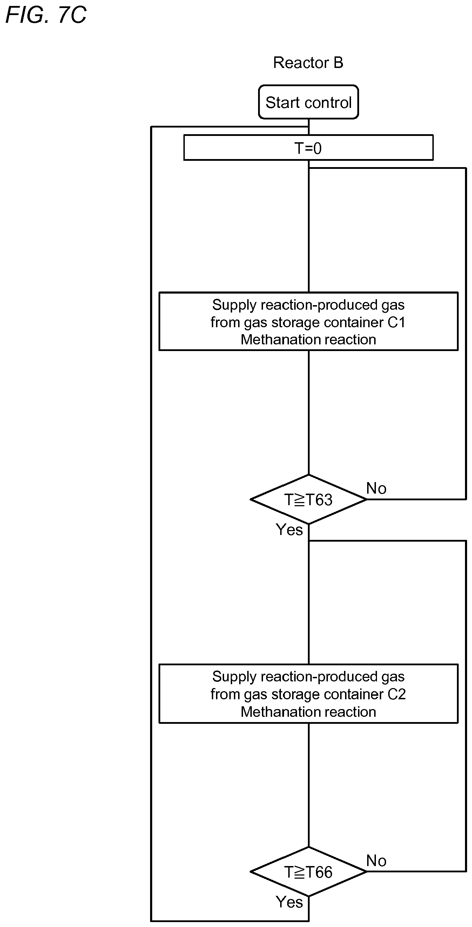

Methane was continuously produced with a gas containing CO₂ and O₂ as a raw material by using the apparatus of producing methane illustrated in FIG. 3 and in accordance with the flowcharts described in FIG. 7A to FIG. 7C and with the time settings described in Table 2. In the apparatus of producing methane illustrated in FIG. 3, two reactors A1 and A2 provided with a CO₂ storage-reduction catalyst were disposed in parallel. The gas inlet of each of the reactors A1 and A2 was connected to the gas supply line 3 for supplying a CO₂-containing gas. The gas supply line 3 was connected to the means 1 for supplying purge gas and the means 2 for supplying reducing gas. In addition, the gas outlet of each of the reactors A1 and A2 was connected to the gas transfer line 4 for transferring the obtained reaction-produced gas to the reactor B provided with a methanation catalyst. The gas storage containers C1 and C2 were disposed on the gas transfer lines 4. Moreover, the gas outlet of the reactor A1 and the gas inlet of the reactor A2 were connected together via the purge gas recirculation line 5. Simultaneously, the gas outlet of the reactor A2 and the gas inlet of the reactor A1 were connected together via the purge gas recirculation line 5.

The CO₂ storage-reduction catalyst, the methanation catalyst, the CO₂-containing gas, the purge gas, and the reducing gas used were the same as those used in Example 1. In addition, the same conditions as those in Example 1 were set for the temperatures of the reactors A1 and A2 and the reactor B and the flow rates of the CO₂-containing gas, the purge gas, and the reducing gas.

**[Table 2]**

| Time [sec] | Reactor A1 | Reactor A2 | Gas Storage Container C1 | Gas Storage Container C2 | Reactor B |
|---|---|---|---|---|---|
| 7.5 | T11 | T21 | --- | T51 | --- |
| 140.5 | --- | T22 | --- | T52 | --- |
| 148.0 | T13 | T23 | T43 | T53 | T63 |
| 155.5 | T14 | T24 | T44 | --- | --- |
| 288.5 | T15 | --- | T45 | --- | --- |
| 296.0 | T16 | T26 | T46 | T56 | T66 |

As shown in FIG. 7A to FIG. 7C and Table 2, the reactor A1 after the reduction reaction (excluding the first cycle) was supplied with the CO₂-containing gas for 7.5 seconds, and simultaneously, the reactor A2 after the storage of CO₂ (excluding the first cycle) was supplied with the purge gas for 7.5 seconds. In addition, in this period of time (for 7.5 seconds), the purge gas emitted from the reactor A2 was supplied to the reactor A1 via the purge gas recirculation lines 5. This allowed the reactor A1 to store CO₂ into the CO₂ storage-reduction catalyst and allowed the reactor A2 to remove reaction-inhibiting components such as O₂. Moreover, in this period of time, a methane-containing gas was obtained (excluding the first cycle) by supplying the reactor B with the reaction-produced gas stored in the gas storage container C1 and reducing the residual CO₂ in the reactor B for generation of methane. Note that the gas storage container C2 was closed in this period of time (for 7.5 seconds).

Next, methane was generated (excluding the first cycle) by supplying the reactor A2 with the reducing gas for 133 seconds to reduce the CO₂ stored in the CO₂ storage-reduction catalyst. The obtained reaction-produced gas (considered to contain methane as well as CO₂ desorbed from the CO₂ storage-reduction catalyst and an unreacted reducing gas) was supplied to the gas storage container C2. After that, the reactor A2 was supplied with the purge gas for 7.5 seconds followed by emission as it was. In this period of time (for 7.5 seconds), the gas storage container C2 was closed and stored the reaction-produced gas. On the other hand, in this period of time (140.5 seconds), the reactor A1 was supplied only with the CO₂-containing gas to store CO₂ in the CO₂ storage-reduction catalyst. In addition, a methane-containing gas was obtained (excluding the first cycle) by subsequently supplying the reactor B with the reaction-produced gas stored in the gas storage container C1 and reducing the residual CO₂ in the reactor B for generation of methane. Note that the supply time taken to supply the reaction-produced gas from the gas storage container C1 to the reactor B was 148 seconds in total (in other words, the reduction time of CO₂ in the reactor B). Next, the reactor A2 was supplied with the CO₂-containing gas for 7.5 seconds, and simultaneously, the reactor A1 after the storage of CO₂ was supplied with the purge gas for 7.5 seconds. In addition, the purge gas emitted from the reactor A1 was supplied to the reactor A2 via the purge gas recirculation lines 5. This allowed the reactor A2 to store CO₂ into the CO₂ storage-reduction catalyst and allowed the reactor A1 to remove the reaction-inhibiting components such as O₂. Moreover, in this period of time, a methane-containing gas was obtained by supplying the reactor B with the reaction-produced gas stored in the gas storage container C2 and reducing the residual CO₂ in the reactor B for generation of methane. Note that the gas storage container C1 was closed in this period of time (for 7.5 seconds).

Next, methane was generated by supplying the reactor A1 with the reducing gas for 133 seconds to reduce the CO₂ stored in the CO₂ storage-reduction catalyst. The obtained reaction-produced gas (considered to contain methane as well as CO₂ desorbed from the CO₂ storage-reduction catalyst and an unreacted reducing gas) was supplied to the gas storage container C1. After that, the reactor A1 was supplied with the purge gas for 7.5 seconds followed by emission as it was. In this period of time (for 7.5 seconds), the gas storage container C1 was closed and stored the reaction-produced gas. On the other hand, in this period of time (140.5 seconds), the reactor A2 was supplied only with the CO₂-containing gas to store CO₂ in the CO₂ storage-reduction catalyst. In addition, a methane-containing gas was obtained by subsequently supplying the reactor B with the reaction-produced gas stored in the gas storage container C2 and reducing the residual CO₂ in the reactor B for generation of methane. Note that the supply time taken to supply the reaction-produced gas from the gas storage container C2 to the reactor B was 148 seconds in total (in other words, the reduction time of CO₂ in the reactor B).

The series of operations described above was repeated until a steady state was reached. A mass spectrometer was used to measure the CO₂ concentrations and methane concentrations in the incoming gas (CO₂-containing gas) and the outgoing gas (methane-containing gas) into/from the apparatus in the steady state. Determined were the amount of CO₂ removed, the amount of CO₂ emitted, and the amount of methane generated per hour based on the obtained measurement results. Table 3 and FIG. 8 to FIG. 10 show the results.

### (Comparative Example 1)

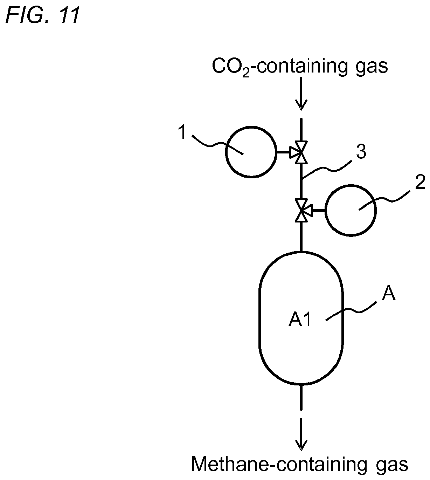

Methane was continuously produced with a gas containing CO₂ and O₂ as a raw material by using the apparatus of producing methane illustrated in FIG. 11. In the apparatus of producing methane illustrated in FIG. 11, one reactor A1 provided with a CO₂ storage-reduction catalyst was disposed. The gas inlet of the reactor A1 was connected to the gas supply line 3 for supplying a CO₂-containing gas. The gas supply line 3 was connected to the means 1 for supplying purge gas and the means 2 for supplying reducing gas.

The CO₂ storage-reduction catalyst, the CO₂-containing gas, the purge gas, and the reducing gas used were the same as those used in Example 1. In addition, the same conditions as those in Example 1 were set for the temperatures of the reactor A1 and the reactor B and the flow rates of the CO₂-containing gas, the purge gas, and the reducing gas.

The reactor A1 after the reduction reaction (excluding the first cycle) was supplied with the CO₂-containing gas for 148 seconds. This allowed the reactor A1 to store CO₂ into the CO₂ storage-reduction catalyst. Next, the reactor A1 after the storage of CO₂ was supplied with the purge gas for 7.5 seconds followed by emission as it was. This allowed the reactor A1 to remove reaction-inhibiting components such as O₂. Next, methane was generated by supplying the reactor A1 with the reducing gas for 133 seconds to reduce the CO₂ stored in the CO₂ storage-reduction catalyst. As a result, a methane-containing gas was obtained. After that, the reactor A1 was supplied with the purge gas for 7.5 seconds followed by emission as it was.

The series of operations described above was repeated until a steady state was reached. A mass spectrometer was used to measure the CO₂ concentrations and methane concentrations in the incoming gas (CO₂-containing gas) and the outgoing gas (methane-containing gas) into/from the apparatus in the steady state. Determined were the amount of CO₂ removed, the amount of CO₂ emitted, and the amount of methane generated per hour based on the obtained measurement results. Table 3 and FIG. 8 to FIG. 10 show the results.

### (Comparative Example 2)

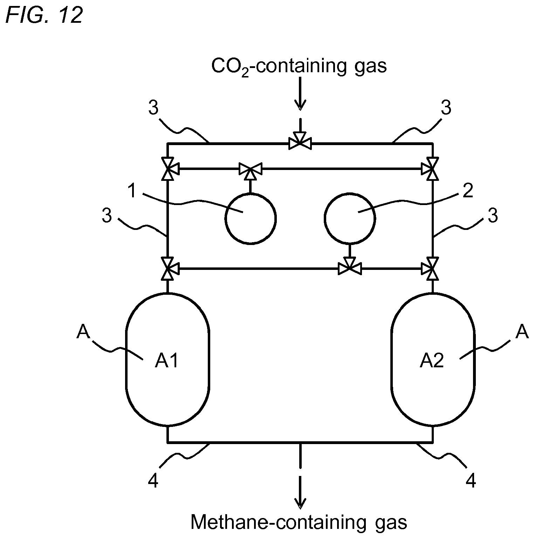

Methane was continuously produced with a gas containing CO₂ and O₂ as a raw material by using the apparatus of producing methane illustrated in FIG. 12. In the apparatus of producing methane illustrated in FIG. 12, two reactors A1 and A2 provided with a CO₂ storage-reduction catalyst were disposed in parallel. The gas inlet of each of the reactors A1 and A2 was connected to the gas supply line 3 for supplying a CO₂-containing gas. The gas supply line 3 was connected to the means 1 for supplying purge gas and the means 2 for supplying reducing gas.

The CO₂ storage-reduction catalyst, the CO₂-containing gas, the purge gas, and the reducing gas used were the same as those used in Example 1. In addition, the same conditions as those in Example 1 were set for the temperatures of the reactors A1 and A2 and the flow rates of the CO₂-containing gas, the purge gas, and the reducing gas.

The reactor A1 after the reduction reaction (excluding the first cycle) was supplied with the CO₂-containing gas for 7.5 seconds, and simultaneously, the reactor A2 after the storage of CO₂ (excluding the first cycle) was supplied with the purge gas for 7.5 seconds. This allowed the reactor A1 to storage CO₂ into the CO₂ storage-reduction catalyst and allowed the reactor A2 to remove reaction-inhibiting components such as O₂.

Next, methane was generated (excluding the first cycle) by supplying the reactor A2 with the reducing gas for 133 seconds to reduce the CO₂ stored in the CO₂ storage-reduction catalyst. As a result, a methane-containing gas was obtained. After that, the reactor A2 was supplied with the purge gas for 7.5 seconds followed by emission as it was. On the other hand, in this period of time (140.5 seconds), the reactor A1 was supplied only with the CO₂-containing gas to store CO₂ in the CO₂ storage-reduction catalyst.

Next, the reactor A2 was supplied with the CO₂-containing gas for 7.5 seconds, and simultaneously, the reactor A1 after the storage of CO₂ was supplied with the purge gas for 7.5 seconds. This allowed the reactor A2 to store CO₂ into the CO₂ storage-reduction catalyst and allowed the reactor A1 to remove the reaction-inhibiting components such as O₂.

Next, methane was generated by supplying the reactor A1 with the reducing gas for 133 seconds to reduce the CO₂ stored in the CO₂ storage-reduction catalyst. As a result, a methane-containing gas was obtained. After that, the reactor A1 was supplied with the purge gas for 7.5 seconds followed by emission as it was. On the other hand, in this period of time (140.5 seconds), the reactor A2 was supplied only with the CO₂-containing gas to store CO₂ in the CO₂ storage-reduction catalyst.

The series of operations described above was repeated until a steady state was reached. A mass spectrometer was used to measure the CO₂ concentrations and methane concentrations in the incoming gas (CO₂-containing gas) and the outgoing gas (methane-containing gas) into/from the apparatus in the steady state. Determined were the amount of CO₂ removed, the amount of CO₂ emitted, and the amount of methane generated per hour based on the obtained measurement results. Table 3 and FIG. 8 to FIG. 10 show the results.

**[Table 3]**

| [mmol/hr] | Example 1 | Example 2 | Comparative Example 1 | Comparative Example 2 |
|---|---|---|---|---|
| Amount of CO₂ removed | 2.68 | 2.68 | 1.13 | 2.26 |
| Amount of CO₂ emitted | 0 | 0 | 0.21 | 0.42 |
| Amount of methane generated | 2.24 | 2.64 | 0.80 | 1.60 |

As shown in Table 3 and FIG. 8 to FIG. 10, each of the amount of CO₂ removed and the amount of methane generated doubled in the case of continuously producing methane by disposing two reactors provided with the CO₂ storage-reduction catalyst in parallel (Comparative Example 2) compared to the case of alternately storing and reducing CO₂ by using one reactor provided with the CO₂ storage-reduction catalyst (Comparative Example 1). However, since the amount of the purge gas emitted also doubled, the amount of CO₂ emitted doubled, too.

On the other hand, even in comparison with Comparative Example 2, the amount of CO₂ removed and the amount of methane generated increased, and furthermore, no emission of CO₂ was detected in the cases (Examples 1 and 2) of using the apparatus of producing methane of the present invention in which the two reactors provided with the CO₂ storage-reduction catalyst are disposed in parallel, their gas inlets and gas outlets are connected to each other via purge gas recirculation lines, and the reactor provided with the methanation catalyst is disposed downstream of the reactors provided with the CO₂ storage-reduction catalyst on the gas flow paths. In addition, as is obvious from the results of Examples 1 and 2, it was found that the amount of methane generated increased by disposing the gas storage container between the reactors provided with the CO₂ storage-reduction catalyst and the reactor provided with a methanation catalyst.

As described above, the present invention makes it possible to prevent emission of CO₂ at the time of removing reaction-inhibiting components such as O₂ contained in a CO₂-containing gas and further to enhance the concentration and the purity of methane in the methane-containing gas to be obtained.

Therefore, the apparatus and the method for producing methane of the present invention are useful as e.g. an apparatus and a method which make it possible to continuously produce methane using, as a raw material, a gas such as a combustion exhaust gas or a biogas which contains CO₂ and reaction-inhibiting components such as O₂.

### Reference Signs List

A, A1, A2: a reactor provided with a CO₂ storage-reduction catalyst
B: a reactor provided with a methanation catalyst
C, C1, C2: a gas storage container
1: a means for supplying purge gas
2: a means for supplying reducing gas
3: a gas supply line
4: a gas transfer line
5: a purge gas recirculation line
6: a means for removing moisture in gas

## Claims

1. An apparatus of producing methane from a CO₂-containing gas, comprising:
at least two reactors (A) which are provided with a CO₂ storage-reduction catalyst in which a component having a methanation catalytic ability and a component having a CO₂ storage capacity are supported on a catalytic support;
at least one reactor (B) which is provided with a methanation catalyst in which a methanation catalytic component is supported on a porous catalytic support;
at least one means (1) for supplying purge gas; and
at least one means (2) for supplying reducing gas, wherein
two or more of the reactors (A) provided with the CO₂ storage-reduction catalyst are connected in parallel,
the at least one means (1) for supplying purge gas selected from the group consisting of noble gases and inert gases and the means (2) for supplying reducing gas are disposed upstream of the reactors (A) provided with the CO₂ storage-reduction catalyst on gas flow paths,
the reactor (B) provided with the methanation catalyst is disposed downstream of the reactors (A) provided with the CO₂ storage-reduction catalyst on the gas flow paths,
a gas supply line (3) for supplying the CO₂-containing gas and the means (2) for supplying reducing gas are connected to each one of the reactors (A) provided with the CO₂ storage-reduction catalyst such that the CO₂-containing gas and the reducing gas are separately and independently supplied to the each one of the reactors (A) provided with the CO₂ storage-reduction catalyst,
a gas outlet of each one of the reactors (A) provided with the CO₂ storage-reduction catalyst is connected to a gas inlet of at least one different reactor (A) provided with the CO₂ storage-reduction catalyst, other than the one reactor, via a purge gas recirculation line (5) which supplies the purge gas containing CO₂ emitted from the gas outlet of the one reactor into the gas inlet of the different reactor,
each one of the reactors provided with the CO₂ storage-reduction catalyst has a feature capable of alternately carrying out the storing of CO₂ and the reducing of CO₂, and
at least one of the reactors provided with the CO₂ storage-reduction catalyst is configured for carrying out the storage of CO₂ and simultaneously a remaining reactor provided with the CO₂ storage-reduction catalyst is configured for carrying out the reducing of CO₂.

2. The apparatus according to claim 1, wherein
at least one gas storage container (C), which stores a reaction-produced gas emitted from gas outlets of the reactors (A) provided with the CO₂ storage-reduction catalyst, is further disposed between the reactors (A) provided with the CO₂ storage-reduction catalyst and the reactor (B) provided with the methanation catalyst.

3. The apparatus according to claim 2, wherein
the gas storage container (C) is disposed between the reactor (B) provided with the methanation catalyst and each of the reactors (A) provided with the CO₂ storage-reduction catalyst.

4. A method for producing methane from a CO₂-containing gas using the apparatus of producing methane according to any one of claims 1 to 3, the method comprising the steps of:
allowing the CO₂ storage-reduction catalyst to store CO₂ by supplying a CO₂-containing gas to one of the reactors (A) provided with the CO₂ storage-reduction catalyst;
reducing the CO₂ by supplying a reducing gas to the one reactor (A) provided with the CO₂ storage-reduction catalyst having stored the CO₂;
supplying a reaction-produced gas obtained by reduction of the CO₂ to the reactor (B) provided with the methanation catalyst;
supplying at least one purge gas selected from the group consisting of noble gases and inert gases to the one reactor (A) after the storage of the CO₂ and supplying the purge gas containing CO₂ emitted from the one reactor to the one reactor after the reduction reaction to allow the CO₂ storage-reduction catalyst to store the CO₂ which is contained in the purge gas; and
reducing residual CO₂ in the reaction-produced gas supplied to the reactor (B) provided with the methanation catalyst, wherein
each one of the reactors (A) provided with the CO₂ storage-reduction catalyst alternately carries out the storing of CO₂, the supplying of the purge gas and the reducing of CO₂, and
at least one of the reactors (A) provided with the CO₂ storage-reduction catalyst carries out the storage of CO₂ and simultaneously a remaining reactor (A) provided with the CO₂ storage-reduction catalyst carries out the reducing of CO₂.

5. The method for producing methane from a CO₂-containing gas according to claim 4 using the apparatus of producing methane according to claim 2 or 3, wherein
the reaction-produced gas obtained by reduction of CO₂ is stored in the gas storage container (C) and then is supplied to the reactor (B) provided with the methanation catalyst.

## Patentansprüche

1. Vorrichtung zum Produzieren von Methan aus CO₂-haltigem Gas, mit:
mindestens zwei Reaktoren (A), die mit einem CO₂-Speicherreduktionskatalysator versehen sind, in dem eine Komponente, die eine Methanisierungskatalysefähigkeit aufweist, und eine Komponente, die ein CO₂-Speichervermögen aufweist, auf einem katalytischen Träger getragen werden;
mindestens einem Reaktor (B), der mit einem Methanisierungskatalysator versehen ist, in dem eine Methanisierungskatalysekomponente auf einem porösen katalytischen Träger getragen wird;
mindestens einem Mittel (1) zum Zuführen von Spülgas; und
mindestens einem Mittel (2) zum Zuführen von Reduktionsgas, bei der zwei oder mehr der Reaktoren (A), die mit dem CO₂-Speicherreduktionskatalysator versehen sind, parallel verbunden sind,
das mindestens eine Mittel (1) zum Zuführen von Spülgas, das aus der Gruppe ausgewählt ist, die aus Edelgasen und Inertgasen besteht, und das Mittel (2) zum Zuführen von Reduktionsgas stromaufwärts der Reaktoren (A), die mit dem CO₂-Speicherreduktionskatalysator versehen sind, auf Gasströmungswegen angeordnet sind,
der Reaktor (B), der mit dem Methanisierungskatalysator versehen ist, stromabwärts der Reaktoren (A), die mit dem CO₂-Speicherreduktionskatalysator versehen sind, auf den Gasströmungswegen angeordnet ist,
eine Gaszufuhrleitung (3) zum Zuführen des CO₂-haltigen Gases und das Mittel (2) zum Zuführen von Reduktionsgas mit jedem der Reaktoren (A), die mit dem CO₂-Speicherreduktionskatalysator versehen sind, verbunden sind, so dass das CO₂-haltige Gas und das Reduktionsgas separat und unabhängig jedem der Reaktoren (A), die mit dem CO₂-Speicherreduktionskatalysator versehen sind, zugeführt werden,
ein Gasauslass jedes der Reaktoren (A), die mit dem CO₂-Speicherreduktionskatalysator versehen sind, mit einem Gaseinlass mindestens eines verschiedenen anderen Reaktors (A), der mit dem CO₂-Speicherreduktionskatalysator versehen ist, als dem einen Reaktor über eine Spülgasrückführungsleitung (5), die das Spülgas, das CO₂ enthält, das von dem Gasauslass des einen Reaktors ausgestoßen wird, in den Gaseinlass des verschiedenen Reaktors zuführt, verbunden ist,
jeder der Reaktoren, die mit dem CO₂-Speicherreduktionskatalysator versehen sind, ein Merkmal aufweist, dass er imstande ist, alternierend die Speicherung von CO₂ und das Reduzieren von CO₂ auszuführen, und
mindestens einer der Reaktoren, die mit dem CO₂-Speicherreduktionskatalysator versehen sind, zum Ausführen der Speicherung von CO₂ ausgebildet ist, und simultan ein verbleibender Reaktor, der mit dem CO₂-Speicherreduktionskatalysator versehen ist, zum Ausführen der Reduzierung von CO₂ ausgebildet ist.

2. Vorrichtung nach Anspruch 1, bei der
mindestens ein Gasspeicherbehälter (C), der ein reaktionserzeugtes Gas, das von Gasauslässen der Reaktoren (A), die mit dem CO₂-Speicherreduktionskatalysator versehen sind, ausgestoßen wird, speichert, ferner zwischen den Reaktoren (A), die mit dem CO₂-Speicherreduktionskata-lysator versehen sind, und dem Reaktor (B), der mit dem Methanisierungskatalysator versehen ist, angeordnet ist.

3. Vorrichtung nach Anspruch 2, bei der
der Gasspeicherbehälter (C) zwischen dem Reaktor (B), der mit dem Methanisierungskatalysator versehen ist, und jedem der Reaktoren (A), die mit dem CO₂-Speicherreduktionskatalysator versehen sind, angeordnet ist.

4. Verfahren zum Produzieren von Methan aus CO₂-haltigem Gas unter Verwendung der Vorrichtung zum Produzieren von Methan nach einem der Ansprüche 1 bis 3, mit den Schritten:
Zulassen, dass der CO₂-Speicherreduktionskatalysator CO₂ speichert, indem ein CO₂-haltiges Gas einem der Reaktoren (A), die mit dem CO₂-Speicherreduktionskatalysator versehen sind, zugeführt wird;
Reduzieren des CO₂ durch Zuführen eines Reduktionsgases zu dem einen Reaktor (A), der mit dem CO₂-Speicherreduktionskatalysator, der das CO₂ gespeichert hat, versehen ist;
Zuführen eines reaktionserzeugten Gases, das durch Reduktion des CO₂ erhalten wird, zu dem Reaktor (B), der mit dem Methanisierungskatalysator versehen ist;
Zuführen mindestens eines Spülgases, das aus der Gruppe ausgewählt wird, die aus Edelgasen und Inertgasen besteht, zu dem einen Reaktor (A) nach der Speicherung des CO₂ und Zuführen des Spülgases, das CO₂ enthält, das von dem einen Reaktor ausgestoßen wird, zu dem einen Reaktor, nach der Reduktionsreaktion, so dass zugelassen wird, dass der CO₂-Speicherreduktions-katalysator das CO₂, das in dem Spülgas enthalten ist, speichert; und
Reduzieren von übrigem CO₂ in dem reaktionserzeugten Gas, das dem Reaktor (B), der mit dem Methanisierungskatalysator versehen ist, zugeführt wird, bei dem
jeder der Reaktoren (A), die mit dem CO₂-Speicherreduktionskatalysator versehen sind, alternierend die Speicherung von CO₂, das Zuführen des Spülgases und das Reduzieren von CO₂ ausführt, und
mindestens einer der Reaktoren (A), die mit dem CO₂-Speicherreduktionskatalysator versehen sind, die Speicherung von CO₂ ausführt, und simultan ein verbleibender Reaktor (A), der mit dem CO₂-Speicherreduktionskatalysator versehen ist, das Reduzieren von CO₂ ausführt.

5. Verfahren zum Produzieren von Methan aus CO₂-haltigem Gas nach Anspruch 4 unter Verwendung der Vorrichtung zum Produzieren von Methan nach Anspruch 2 oder 3, bei dem das reaktionserzeugte Gas, das durch Reduktion von CO₂ erhalten wird, in dem Gasspeicherbehälter (C) gespeichert wird und dann dem Reaktor (B), der mit dem Methanisierungskatalysator versehen ist, zugeführt wird.

## Revendications

1. Appareil de production de méthane à partir d'un gaz contenant du CO₂, comprenant :
au moins deux réacteurs (A) qui sont pourvus d'un catalyseur de réduction de stockage de CO₂ dans lequel un composant ayant une capacité catalytique de méthanation et un composant ayant une capacité de stockage de CO₂ sont supportés sur un support catalytique ;
au moins un réacteur (B) qui est pourvu d'un catalyseur de méthanation dans lequel un composant catalytique de méthanation est supporté sur un support catalytique poreux ;
au moins un moyen (1) pour fournir un gaz de purge ; et
au moins un moyen (2) pour fournir un gaz réducteur, dans lequel
deux ou plusieurs des réacteurs (A) munis du catalyseur de réduction de stockage de CO₂ sont connectés en parallèle,
ledit au moins un moyen (1) d'alimentation en gaz de purge choisi dans le groupe constitué de gaz nobles et les gaz inertes et le moyen (2) d'alimentation en gaz de réduction sont disposés en amont des réacteurs (A) munis du catalyseur de réduction de stockage de CO₂ sur des voies d'écoulement de gaz,
le réacteur (B) pourvu du catalyseur de méthanation est disposé en aval des réacteurs (A) pourvus du catalyseur de réduction de stockage de CO₂ sur les voies d'écoulement de gaz,
une ligne d'alimentation en gaz (3) pour fournir le gaz contenant du CO₂ et le moyen (2) pour fournir le gaz de réduction sont connectés à chacun des réacteurs (A) pourvus du catalyseur de réduction de stockage de CO₂ de sorte que le gaz contenant du CO₂ et le gaz de réduction sont fournis séparément et indépendamment à chacun des réacteurs (A) pourvus du catalyseur de réduction de stockage de CO₂,
une sortie de gaz de chacun des réacteurs (A) pourvus du catalyseur de réduction de stockage de CO₂ est reliée à une entrée de gaz d'au moins un réacteur différent (A) pourvu du catalyseur de réduction de stockage de CO₂, autre que le premier réacteur, par l'intermédiaire d'une ligne de recirculation de gaz de purge (5) qui fournit le gaz de purge contenant du CO₂ émis par la sortie de gaz du premier réacteur dans l'entrée de gaz du réacteur différent,
chacun des réacteurs pourvus du catalyseur de réduction de stockage de CO₂ a une caractéristique capable de réaliser alternativement le stockage de CO₂ et la réduction de CO₂, et
au moins un des réacteurs pourvus du catalyseur de réduction de stockage de CO₂ est configuré pour réaliser le stockage de CO₂ et simultanément un réacteur restant pourvu du catalyseur de réduction de stockage de CO₂ est configuré pour réaliser la réduction de CO₂.

2. Appareil selon la revendication 1, dans lequel
au moins un récipient de stockage de gaz (C), qui stocke un gaz produit par réaction émis par les sorties de gaz des réacteurs (A) munis du catalyseur de réduction de stockage de CO₂, est en outre disposé entre les réacteurs (A) munis du catalyseur de réduction de stockage de CO₂ et le réacteur (B) muni du catalyseur de méthanation.

3. Appareil selon la revendication 2, dans lequel
le récipient de stockage de gaz (C) est disposé entre le réacteur (B) muni du catalyseur de méthanation et chacun des réacteurs (A) munis du catalyseur de réduction de stockage de CO₂.

4. Procédé de production de méthane à partir d'un gaz contenant du CO₂ en utilisant l'appareil de production de méthane selon l'une quelconque des revendications 1 à 3, le procédé comprenant les étapes consistant à :
permettre au catalyseur de réduction de stockage de CO₂ de stocker du CO₂ en fournissant un gaz contenant du CO₂ à l'un des réacteurs (A) équipé du catalyseur de réduction de stockage de CO₂;
réduire le CO₂ en fournissant un gaz réducteur à un des réacteurs (A) pourvu du catalyseur de réduction de stockage de CO₂ ayant stocké le CO₂ ;
fournir un gaz produit par la réaction, obtenu par réduction du CO₂, au réacteur (B) pourvu du catalyseur de méthanation ;
fournir au moins un gaz de purge choisi dans le groupe constitué de gaz nobles et de gaz inertes au premier réacteur (A) après le stockage du CO₂ et fournir le gaz de purge contenant le CO₂ émis par le premier réacteur au premier réacteur après la réaction de réduction pour permettre au catalyseur de réduction de stockage de CO₂ de stocker le CO₂ qui est contenu dans le gaz de purge ; et
réduire le CO₂ résiduel dans le gaz produit par la réaction fourni au réacteur (B) muni du catalyseur de méthanation, dans lequel
chacun des réacteurs (A) pourvus du catalyseur de réduction de stockage de CO₂ effectue alternativement le stockage du CO₂, la fourniture du gaz de purge et la réduction du CO₂, et
au moins un des réacteurs (A) pourvus du catalyseur de réduction de stockage de CO₂ effectue le stockage de CO₂ et simultanément un réacteur restant (A) pourvu du catalyseur de réduction de stockage de CO₂ effectue la réduction de CO₂.

5. Procédé de production de méthane à partir d'un gaz contenant du CO₂ selon la revendication 4, utilisant l'appareil de production de méthane selon la revendication 2 ou 3, dans lequel
le gaz produit par la réaction obtenu par réduction du CO₂ est stocké dans le récipient de stockage de gaz (C) puis est fourni au réacteur (B) muni du catalyseur de méthanation.
